# EUROPEAN PATENT SPECIFICATION

(11) **EP 1 436 216 B1**
(45) Date of publication and mention of the grant of the patent: **15.11.2006**
(21) Application number: 02782870.6
(22) Date of filing: 09.10.2002
(51) Int. Cl.: B65D 83/04, A61M 15/00

(54) **MEDICAMENT DISPENSER**
ABGABEVORRICHTUNG FÜR ARZNEIMITTEL
DISTRIBUTEUR DE MEDICAMENTS

(30) Priority: 19.10.2001 GB 0125135
(43) Date of publication of application: 14.07.2004
(73) Proprietor: GLAXO GROUP LIMITED, Greenford, Middlesex UB6 ONN (GB)
(72) Inventor: HARVEY, Stephen, James c/o Glaxo Wellcome plc, Ware Herts SG12 ODP (GB)
(74) Representative: Rice, Jason Neale
(86) International application number: PCT/EP2002/011312
(87) International publication number: WO 2003/035508

(56) References cited:
- WO-A-01/41846
- WO-A-01/66061
- DE-A- 3 210 361
- US-A- 5 873 360
- "LE DISTRIBUTEUR A RUBAN DISKUS" ANNALES FRANCAISES DE CHRONOMETRIE ET DE MICROTECHNIQUE, OBSERVATOIRE DE BESANCON. BESANCON, FR, vol. 47, 1998, pages 97-105, XP000830919 ISSN: 0294-1228

## Description

### Technical field

The present invention relates to a medicament dispenser for dispensing medicament. The invention particularly relates to a device for use in dispensing medicament in powder or tablet form.

### Background to the invention

The use of inhalation devices in the administration of medicaments, for example in bronchodilation therapy is well known. Such devices generally comprise a body or housing within which a medicament carrier is located. Known inhalation devices include those in which the medicament carrier is a blister strip containing a number of discrete doses of powdered medicament. Such devices usually contain a mechanism for accessing these doses, usually comprising either piercing means or means to peel a lid sheet away from a base sheet. The powdered medicament can then be accessed and inhaled. Such a mechanism may also be used for dispensing medicament in tablet form wherein peeling away the lid sheet from the base sheet reveals a tablet for removal and subsequent consumption.

It is an object of the present invention to simplify the internal mechanism of a medicament dispenser for dispensing medicament in powder or solid form from a medicament carrier as described *supra.*

Yet another object of the present invention is to provide a device that is refillable by insertion of a replacement cassette containing a medicament carrier. The cassette may be replaced when the medicament carrier is empty. The device is therefore more 'environmentally friendly' as the majority of the device may be retained and is not disposable. It also allows the device to be fitted with additional features such as electronics which may not be cost effective on a completely disposable device.

It is a further object of the present invention that the cassette may be easily removed and that a new replacement cassette can be easily inserted. It is also desirable that the operation of the medicament dispenser be straightforward and non-complex and in particular that the number of separate steps involved in preparing the device for use be minimised. This is especially relevant where the device is designed for use in the delivery of medicament in emergency or rescue situations (e.g. asthma attacks) where simplicity and ease of use is paramount.

When not in use it is desirable from a hygiene standpoint that a mouthpiece, or other medicament exit channel, is provided with some kind of protective cover. The cover desirably acts both to prevent buitd-up of dirt and to prevent ingress of dirt into the body of the device through the mouthpiece or channel, which might then be subject to inhalation or consumption by a patient. It is also desirable that the cover is in some way attached or mounted to the device to minimise the risk that the cover is misplaced or lost. It is therefore a further object of the present invention for the body of the device to act as a mouthpiece or exit channel cover when the device is in storage and that the cassette is movable relative to the body to enable the mouthpiece or channel to be uncovered for use by the patient.

It is a further object of the invention to provide a medicament dispenser device suitable for use with a large number of discrete doses but which is of an acceptable size for use by patients.

A medicament dispenser according to the pre-characterising part of claim 1 hereof is known from "LE DISTRIBUTEUR A RUBAN DISKUS", Annales Francaises de Chronometrie et de Microtechnique, Observatoire de Besancon, Besancon, FR, vol. 47, 1998, p.97-105, XP000919 ISSN:0294-1228.

### Summary of the invention

Accordingly, the invention provides a medicament dispenser for use with a medicament carrier having a plurality of pockets for containing medicament wherein said pockets are spaced along the length of and defined between two peelable sheets secured to each other, said dispenser having an internal mechanism for accessing said medicament contained within said medicament carrier, said mechanism comprising,
a) an opening station for receiving a pocket of said medicament carrier;
b) peeling means positioned to engage a base sheet and a lid sheet of a pocket which has been received in said opening station for peeling apart such a base sheet and lid sheet, to open such a pocket, said peeling means including lid driving means for pulling apart a lid sheet and a base sheet of a pocket that has been received at said opening station;
c) an outlet, positioned to be in communication with an opened pocket through which a user can remove medicament from such an opened pocket; and
d) indexing means for indexing in communication with said outlet, pockets of a medicament carrier in use with said medicament dispenser,
wherein said indexing means comprises an index ratchet which is movable between a locked position in which said ratchet engages said medicament carrier to prevent movement thereof and a release position in which the ratchet disengages from the medicament carrier to allow movement thereof for peeling open of a blister pocket.

In use, actuation of said medicament dispenser actuates said lid driving means and reversibly disengages said index ratchet from said medicament carrier to allow peeling thereof (e.g. by enabling free movement of the medicament carrier). Suitably, the actuation of the lid driving means and release of the index ratchet are coupled.

In the present invention, the lid driving means acts such as to draw the medicament carrier through the internal accessing mechanism, and in particular to draw the carrier through the opening station for peelable opening thereof. In use, it is generally desirable that a single pocket (or other pre-determined number of pockets) is opened and that further opening is prevented. Herein, the index ratchet provides the means for achieving this desired function.

In use, the index ratchet is initially positioned ('locked position') such as to prevent travel of the medicament carrier. In response to actuation of the dispenser however, the lid driving means is actuated and the index ratchet reversibly released ('release position') to enable a defined degree of travel of the medicament carrier to the opening station. The defined degree of travel corresponds generally to that required to move one blister pocket to the opening station and open the pocket for release of a dose (generally, one pocket's worth) of medicament. Once the defined degree of travel has been undertaken, the index ratchet again locks ('locked position') and thereby acts such as to prevent further movement of the medicament carrier and hence further peeling thereof, until after the released dose of medicament has been dispensed to the patient.

The index ratchet may have any suitable form. In aspects, it comprises a ratchet arm that is suitably, pivotally mounted on the dispenser. The ratchet arm is generally shaped (e.g. with a dog-leg end) to engage blister pockets of the medicament carrier and located within the medicament dispenser to ensure that such engagement occurs in ratchet fashion.

Suitably, in the locked position the index ratchet engages a pocket on said medicament carrier and in the release position said pocket is disengaged. When the pocket is engaged the medicament carrier is prevented from moving within the dispenser, but when dis-engaged the medicament carrier is movable within the dispenser for peelable opening of a pocket thereof.

Suitably, the index ratchet is positioned to cam in and out of engagement with a blister pocket of the medicament carrier. In aspects, the index ratchet cams in and out to locate directly behind the blister pocket of a medicament carrier, thereby providing consistent (single) pocket feed to the opening station.

Suitably, the medicament dispenser further comprises an indexing lever for actuating said dispenser (i.e to actuate the lid driving means and release the index ratchet from said medicament carrier to allow peeling thereof).

Typically, said indexing lever comprises moving means (e.g. cam-form) for moving said index ratchet between locked and release positions, such that actuation of said lever from a rest position releases said medicament carrier for peeling thereof.

Typically, said indexing lever also comprises engagement means (e.g. a lever ratchet) for engaging said lid driving means.

Suitably, said lid driving means comprises an index gear and a drive gear which are interconnected so that the rotation of one correlates with the rotation of the other.

Suitably, said lid driving means comprises a wheel on which the lid sheet is wound up.

Typically, said lid sheet wheel has an effective winding surface, the diameter of which increases after every use of the dispenser as the lid sheet winds around the wheel.

In order to ensure that the same dose is dispensed every time, that is, only one medicament pocket is opened for every actuation of the dispenser, the dispenser may further comprise a lever stop means to limit the extent of movement of said index lever and thereby said lid driving means, in order to control the length of medicament carrier peeled by said peeling means. Hence, the medicament carrier is indexed by the same amount each time and a uniform, consistent dose is always dispensed.

In one aspect, the dispenser further comprises compensating means positioned between said opening station and said lid sheet wheel for reducing the length of said lid sheet therebetween to compensate for any increase in the diameter of the effective winding surface of the lid driving means during use of the dispenser.

Typically, the compensating means takes the form of a flexible member. The flexible member may take the form of a flexible elongate arm about which the lid sheet is fed. The arm may flex inwards as tension in the lid sheet increases, and thus shorten the length of lid sheet between the opening station and the lid driving means.

Suitably, the compensating means takes the form of a spring which reduces in length as tension. increase in the lid sheet between the opening station and the lid driving means. Typically a piston head is mounted on one end of the spring about which the lid sheet is fed. The other end of the spring may be fixed. As tension in the lid sheet increases the piston is driven down onto the spring.

Suitably, the compensating means takes the form of a sprung-loaded tensioner.

Suitably, the flexible member is resilient so that on removal of tension from the lid sheet, the flexible member will return to its rest position. Thus, the internal mechanism can be reloaded with a new medicament carrier after the used carrier is removed.

In another aspect, the dispenser comprises a clutch means to adjust for any increase in the diameter of the effective winding surface of the lid driving means during use of the dispenser. In one aspect, the clutch means communicates with the indexing means and the lid driving means, and comprises a gearing surface defining plural gear engagement positions; and plural gear teeth for engaging said plural gear engagement positions, wherein the plural gear teeth are arranged such that at any one time only a single gear tooth engages a single gear engagement position.

It will be appreciated that, in use, the clutch means acts to compensate for the increase in diameter of said effective winding surface of the lid driving means. The clutch means allows for slippage when the tension in the lid sheet is greater than the force required to peel apart the lid sheet and the base sheet.

It will be appreciated that in total, the clutch means effectively defines a number of individual gear positions which is greater than the number of gear engagement positions. This is therefore advantageous over a traditional slipping clutch arrangement comprising intermeshing gear wheels, where the effective number of individual gear positions defined is either equal to, or no more than, the number of gear engagement positions defined by one of the gear wheels. The clutch means herein is also typically more compact than traditional slipping clutch arrangements e.g. because it enables smaller gearing surfaces to be employed.

Suitably, the gearing surface and plural gear teeth are arranged such that the number of individual gear positions defined is equal to the number of gear engagement positions multiplied by the number of gear teeth. In one example, if the gearing surface defines 60 gear engagement positions and there are 6 gear teeth, then up to 360 individual gear positions are definable (e.g. 1° resolution on a rotating gear system).

Suitably, the gearing surface defines from 20 to 100, preferably from 40 to 80 gear engagement positions. Suitably, the number of gear teeth is from 2 to 20, preferably from 3 to 10.

In one aspect, the gear engagement positions are equally spaced (e.g. equidistantly spaced) and the gear teeth are offset (e.g. non-equidistantly spaced) relative thereto. Such offset arrangement maximises the number of effective individual gear positions which are capable of definition. An example of this aspect, is the Vernier spring arrangement described herein.

In another aspect, the gear engagement positions are also equally spaced (e.g. equidistantly spaced) and the gear teeth are located on a wobbling element capable of wobbling the gear teeth to plural offset (e.g. non-equidistantly spaced) positions. Such a wobbling offset arrangement also maximises the number of effective individual gear positions which are capable of being defined. An example of this aspect, is the wobbling wheel arrangement described herein.

In aspects, the clutch means is non-integral with either of the lid driving means or the indexing means, but forms a separate interconnecting component.

Suitably, the gearing surface comprises a gear wheel. As used herein, the term gear wheel encompasses, for example, a wheel, spindle or spool.

Suitably, the gear teeth may be arranged to be in ratchet form (i.e. enabling movement in one direction only).

Suitably, the gearing surface and gear teeth are in biased (e.g. sprung) engagement.

In another aspect, the lid driving means comprises a wheel on which the lid sheet is wound up, said wheel having a winding surface which decreases in diameter when tension in the lid sheet increases.

Suitably, said wheel comprises a plurality of resiliently flexible arms each extending therefrom at an angle with respect to a radius. The leading end of the lid sheet is looped over one of said resiliently flexible arms to secure the lid sheet to the wheel initially.

Alternatively, the lid driving means comprise a mangle. The lid sheet passes through two rotating wheels which act as a mangle and is gripped at the point of contact with the wheels. The used portion of the lid sheet is collected in a chamber after it has passed through the mangle.

Alternatively, the lid driving means comprise a roller. Suitably, said roller is composed of a polymeric rubber and is positioned next to a guide wall. Suitably, said roller has a smooth surface. Alternatively said roller has a knurled surface. The roller grips the lid sheet as it passes from the point at which it is separated from the base sheet through the space between the roller and the guide wall and the used portion of the lid sheet is then collected in a chamber. The roller has the advantage over the mangle described above in that a greater degree of contact between the roller wheel and the lid sheet occurs- the lid sheet is squeezed through the roller and may pass around about 1/3 of the roller wheel. This provides a higher level of grip and pulling force than with a mangle. The force required to turn the roller is constant throughout the use of the device and does not vary according to how much of the lid sheet has been peeled away from the base sheet. This is in contrast to the wheel described above where the forces required to turn the wheel may vary due to the fact that the lid sheet is wound around the wheel. The lid sheet is not wound around the roller. The roller also has the advantage that the lid sheet does not have to be looped around or fixed to the roller before use of the device, therefore simplifying assembly of the device and reducing costs.

In a further aspect, the lid driving means comprise a spiked wheel. As the spiked wheel turns, the lid sheet is pulled over it and the spikes perforate parts of the lid sheet to improve the grip on the lid sheet. The lid sheet then passes out into a chamber where it collects.

In a further aspect, the lid driving means comprise a clamp system. The clamp system comprises at least one angled spring which is pivotable at one end and grips the lid sheet at the other end. The clamp system is moved in the direction that the lid sheet is to be pulled and grips the lid sheet, pulling it and therefore peeling it away from the base sheet. The clamp system is then moved back to its rest position. This results in the spring pivoting and clamping the lid sheet, therefore preventing the lid sheet from being further peeled from the base sheet.

In an alternative aspect, the used portion of the lid sheet may be passed around rollers and fed back onto the used portion of the base sheet after the medicament has been accessed to join back onto the base sheet. The lid sheet may be coated with a sticky substance to aid resealing. The use of this mechanism saves space as the used portions of the blister strip will be collected in the same area.

In a further aspect, the coil comprising the unused medicament strip may be surrounded by a constant force spring. Alternatively the coil comprising the unused medicament strip may be surrounded by an elastomeric band or band comprising a contractible material, The constant force spring, elastomeric band or band comprising a contractible material contracts as the coil reduces in size.

Suitably, said peeling means additionally comprise a guide for guiding the lid sheet and base sheet along separate paths at the opening station. The lid sheet is passed around the guide portion onto the lid driving means.

Suitably, the guide comprises a structure fixed in position in the cassette.

Alternatively, the guide comprises a roller mechanism. The lid sheet is fed over the rollers onto the lid driving means.

In one aspect, the lid driving means and/or the index ratchet are operated by an electronic drive system. The electronic drive system may also be used in conjunction with a mechanical drive system. The electronic drive system may include a DC motor.

Suitably, the internal mechanism additionally comprises a first chamber in which the strip is initially housed and from which it is dispensed and a second chamber to receive the used portion of the base sheet after it has been indexed and separated from the lid sheet.

Suitably, said first chamber and said second chamber are separated by a wall.

Suitably, said wall is movable to adjust the size of said first and second chambers.

Suitably, the wall is pivotally mountable, Alternatively, the wall is slidably mountable.

Suitably, the wall is flexible to allow changes in the relative size of said first and second chambers.

Suitably, the internal mechanism further comprises a third chamber to receive the used portion of the lid sheet and a fourth chamber which houses the index ratchet. The fourth chamber may communicate via a slit, which in turn extends upwardly within a mouthpiece and communicates with air inlets.

Suitably, the internal mechanism additionally comprises a crushing wheel to crush the medicament pockets after the medicament has been removed from them. The crushing wheel therefore reduces the space which the used portion of the base sheet takes up.

Typically, the internal mechanism for accessing said medicament contained within said medicament carrier is housed within a cassette.

The medicament dispenser may comprise a body; a holder, shaped to fit within said body and movable relative to said body; and receivable by said holder, a cassette containing said medicament carrier.

Suitably, movement of the holder relative to the body results in movement of the cassette between a first position and a second position such that the cassette is reversibly removable from the holder when the cassette is in the second position.

Suitably, the first position comprises a dispensing position. Preferably the second position comprises a non-dispensing position. The cassette is therefore only removable from the holder when the cassette is in the non-dispensing position.

Suitably, the holder and body include attaching means to attach the holder to the body. Preferably, said attaching means comprise a snap fit mechanism. Preferably, said snap fit mechanism comprises a pin and hole system.

Suitably, the holder is pivotally movable relative to the body.

Alternatively, the holder is rotationally movable relative to the body.

Suitably, the holder additionally comprises a stop to limit movement of the holder relative to the body. The stop abuts against the edge of the body at two points when it is rotated. At these points the holder may be designed to click into place. Therefore when the stop abuts one body edge then it is clicked into the dispensing position and when the stop abuts the other body edge then it is clicked into the non-dispensing position.

Alternatively, the holder is slidably movable relative to the body.

Suitably, the holder additionally comprises a catch to retain the cassette. The catch may for example comprise a sprung pin which fits into a hole or an integral catch which deforms when pressed allowing removal of the cassette.

Suitably, the catch is child resistant. Child resistance may be realised by having a system which forces the user to perform two actions at once to remove the cassette. Other features of the catch may include shock or impact resistance, the ability to lock the catch and orientation features to ensure that the cassette can only be inserted one way. The catch should also be easy to manufacture and assemble, be robust, be composed of a minimal number of components and intrude minimally into the space into which the cassette is inserted.

Suitably, the holder includes guide means to guide the cassette into the holder. Preferably said guide means comprise guide rails. Alternatively the guide means comprise grooves, indentations or other shaping or surface details to define a 'lock and key' relationship between the holder and the cassette. Colour guides, arrows and any other surface markings may also be employed.

Suitably, the cassette additionally comprises an indexing lever. The indexing lever has a finger tab located outside the body of the cassette. The rest of the indexing lever is located within the cassette. The indexing lever may have teeth at its tail end and/or teeth along its mid portion.

Suitably, the cassette additionally comprises a mouthpiece.

Suitably, said mouthpiece is extendable. The mouthpiece extends as the cassette and holder are moved from the non-dispensing position to the dispensing position.

Alternatively, the mouthpiece is retractable. The mouthpiece retracts as the cassette and holder are moved from the dispensing position to the non-dispensing position.

In one aspect, the mouthpiece is telescopic. Alternatively, the mouthpiece is fixed.

The medicament dispenser may also be designed for nasal inhalation of a powdered medicament and may therefore incorporate a nosepiece as an alternative to a mouthpiece. If the medicament is in solid form, the dispenser may incorporate an exit channel for tablet release.

Suitably, the body covers the mouthpiece and indexing lever when the cassette is in the non-dispensing position. This avoids the need for a separate cover and protects the mouthpiece from the ingress of dirt and contaminants during storage.

Suitably, the cassette additionally comprises a raised portion to fit against the holder. The raised portion is located at the opposite end of the cassette to the mouthpiece/nosepiece/exit and indexing lever and prevents the incorrect insertion of the cassette into the holder since it is too wide to fit into the holder. The raised portion is shaped such that it fits against a cut away part of the holder. Preferably, said raised portion includes a section which is raised to define a grip portion.

Suitably, at least a portion of the holder and body are shaped for ease of grip by the user.

Suitably, operation of the device may be performed with one hand.

Suitably, the medicament dispenser comprises an actuation or dose counter for counting the number of actuations of the indexing lever or releases of dose from the cassette.

The dose counter may count the number of doses left to be taken or the number of doses taken.

Suitably, said dose counter is electronic. Alternatively, said dose counter is mechanical.

Suitably, said dose counter is located within the cassette. Alternatively, the dose counter is external to the cassette.

Alternatively, the blister strip has printed numbers on it corresponding to the doses in the pockets. Preferably, said printed numbers are visible through a window in the cassette.

The device may be assembled as follows. The holder is snap fitted into the body. The cassette is assembled separately. The body of the cassette is formed, preferably in two sections with any necessary spindles or integral components formed into the base. Individual components such as indexing wheels, lid winding mechanisms, guide portions etc are then assembled into the base. Finally the medicament containing blister strip (or other suitable medicament carrier) may be inserted into the cassette. This may be wound into the device before the lid is attached to the cassette and the cassette sealed. Alternatively, the cassette may be formed completely apart from a hole left in its side for insertion of the blister strip or medicament carrier. The hole may then be sealed to complete the cassette. This second method of inserting the medicament carrier into the device has the advantage that it is much simpler.

Suitably, the medicament dispenser additionally comprises an electronic data management system. The electronic data management system has input/output capability and comprises a memory for storage of data; a microprocessor for performing operations on said data; and a transmitter for transmitting a signal relating to the data or the outcome of an operation on the data.

The medicament may comprise a capsule, pellet or tablet. Alternatively, the medicament may be in powdered form. Preferably, when in powdered form the medicament comprises a drug. Preferably the drug is selected from the group consisting of albuterol, salmeterol, fluticasone propionate and beclomethasone dipropionate and salts or solvates thereof and any combination thereof. Preferably said combination comprises salmeterol xinafoate and fluticasone propionate.

Suitably, the powdered medicament additionally comprises an excipient. Suitably, said excipient is a sugar.

### Brief Description of the Drawings

The invention will now be described with reference to the accompanying drawings in which:
Figure 1a shows a cassette housing an internal mechanism in accordance with one aspect of the invention;
Figure 1b shows a base unit of a medicament dispenser onto which the cassette of Figure 1a is receivable,
Figure 2 shows a perspective view of a medicament carrier in accordance with the present invention;
Figure 3a shows an asymmetric cassette comprising an internal mechanism in accordance with another aspect of the invention;
Figure 3b shows a round cassette comprising an internal mechanism in accordance with another aspect of the invention;
Figure 4 shows a perspective view of a medicament dispenser according to the invention with the cassette removed from the holder and the body;
Figure 5a shows a perspective view of a refill cassette comprising an Internal mechanism according to a further aspect of the invention; and
Figure 5b shows the refill cassette of Figure 5a in exploded view.

### Detailed Description of the Drawings

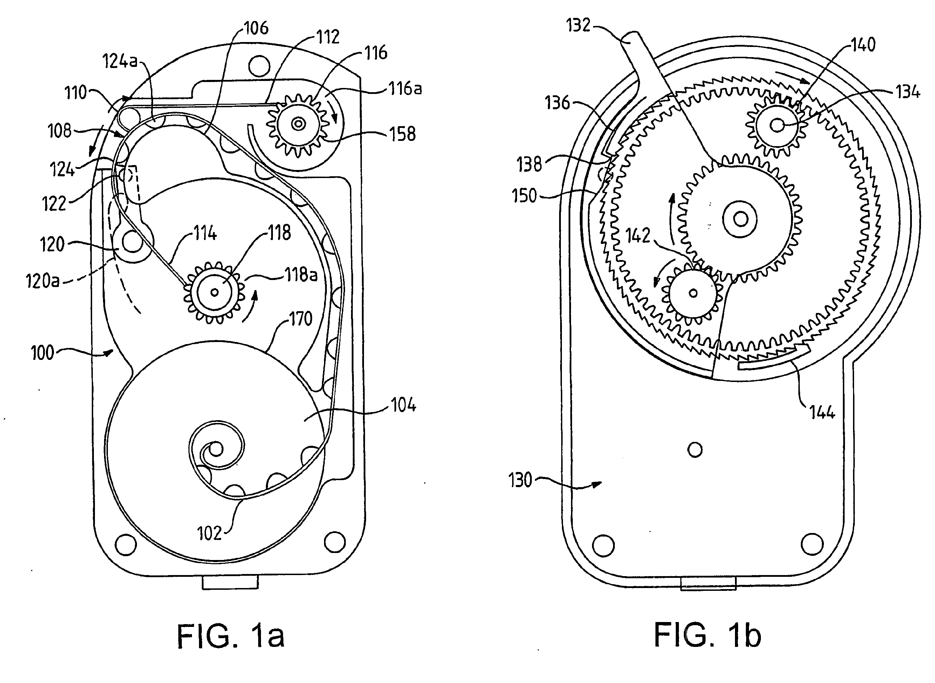

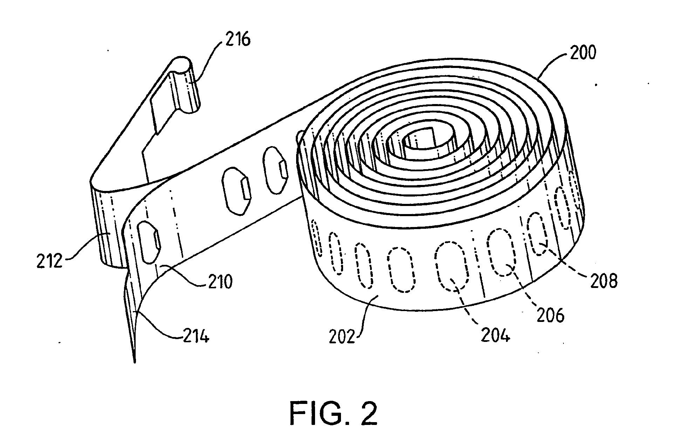

Referring now to the Figures, the internal mechanism according to one aspect of the invention is illustrated housed in a cassette 100 in Figure 1a. The cassette 100 is sized and shaped for receipt by base unit 130 shown in Figure 1b. Medicament carrier in the form of an elongate blister strip 102 (see also Figure 2) is coiled in chamber 104 and fed about a guide wall 106 to the opening station 108 and beak 110. After the blister pack 102 is peeled into separate lid sheet 112 and base sheet 114, the lid sheet 112 is fed to a lid spool 116 in the lid spool chamber 116a, and the base sheet 114 to a base spool 118 in the base spool chamber 118a.

An index ratchet 120 prevents movement of the blister strip 102. A nose 122 on the index ratchet 120 rests adjacent a medicament pocket 124 on the strip 102 hence halting further progression of the strip 102 through the mechanism.

The base unit 130 is shown in Figure 1b and houses the index lever 132 and the lid spool driving means 134. The index lever 132 has a lever ratchet 136 that engages with an index gear 138 which in turn engages and drives the lid spool drive gear 140 and the base spool drive gear 142. Thus, displacement of the index lever 132 results in rotation of the lid spool 116 and base spool 118 in opposite directions to pull apart the lid sheet 112 and the base sheet 114. The lever ratchet 136 and a non-return ratchet 144 prevent movement of the index gear 138 in the other direction. The index lever 132 also has a cam surface 150.

To actuate the medicament dispenser, the index lever 132 is displaced and the pin 120a of the index ratchet 120 follows the cam surface 150 thereof to move the index ratchet 120 away from already-opened pocket 124 of the strip 102 to its 'release position'. In consequence, strip 102 is released for further travel within the dispenser 100. The lid spool driving means 134 is also thereby freed to act such as to peel the lid sheet 112 about beak 110 to open the next pocket 124a to enable the release of medicament therefrom.

As the index lever 132 returns to rest, the cam surface 150 urges the index ratchet 120 back to its 'locked position' whereby the strip 102 is prevented from further movement.

As the lid spool 116 receives the peeled lid sheet 112, the diameter of the outer winding surface 158 gradually increases. In order to ensure that the same dose is dispensed every time, that is, only one blister pack is opened for every actuation of the dispenser, a fever stop (not shown) is fitted which correspondingly shortens the amount the lid spool drive gear 140 is rotated to allow for the increase in diameter of the lid spool winding surface 158. Hence, the medicament carrier is indexed by the same amount each time and a uniform, consistent dose is always dispensed.

Chamber 104 housing the unused blister strip 102 and the base spool chamber 118a, are separated by a wall 170. The wall 170 is movable to adjust the relative size of the chambers 104, 118a, The wall may be pivotally mountable or slidably mountable. In this case, the wall 170 is flexible.

The wall 170 additionally comprises at least one brush (not shown) located along its top or bottom side which brush against the top and bottom surfaces of the inside of the cassette. The brushes may act to close off the chamber from the rest of the body of the cassette and to prevent any loose powder from entering the rest of the cassette. Loose powder may enter the chambers from the used portion of the blister strip if the patient indexes the strip by pressing the lever when they do not intend to take a dose or when they fail to inhale all the powder.

Figure 2 shows a medicament carrier 200 in accord with the present invention. The medicament carrier comprises a flexible strip 202 defining a plurality of pockets 204, 206, 208 each of which contains a dose of medicament which can be inhaled, in the form of powder.

The strip comprises a base sheet 210 in which blisters are formed to define the pockets 204, 206, 208 and a lid sheet 212 which is hermetically sealed to the base sheet except in the region of the blisters in such a manner that the lid sheet 212 and the base sheet 210 can be peeled apart. The sheets 210, 212 are sealed to one another over their whole width except for the leading end portions 214, 216 where they are preferably not sealed to one another at all. The lid 212 and base 210 sheets are each preferably formed of a plastics/aluminium laminate and are preferably adhered to one another by heat sealing.

The strip 202 is shown as having elongate pockets 204, 206, 208 which run transversely with respect to the length of the strip 202. This is convenient in that it enables a large number of pockets 204, 206, 208 to be provided in a given strip length. The strip may, for example, be provided with sixty or one hundred pockets but it will be understood that the strip may have any suitable number of pockets.

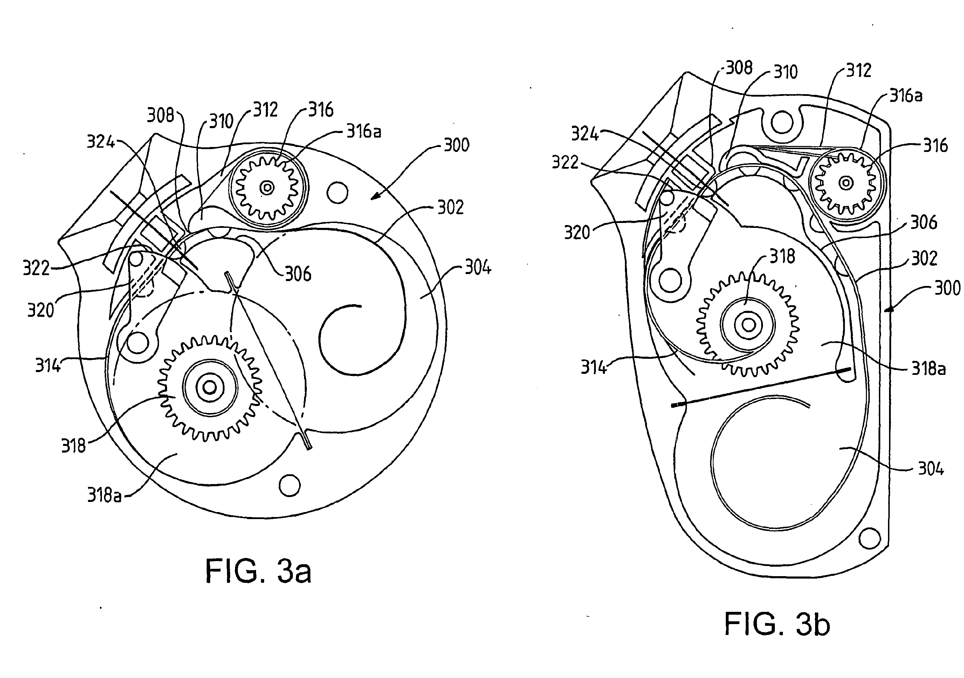

Figures 3a and 3b illustrate variations of the cassette of Figure 1a, in which a corresponding mechanism is accommodated within differently shaped cassette housings. Figure 3b shows an asymmetric variation and Figure 3c shows a round cassette. The cassette housings of Figures 3a and 3b will engage with base units (not shown) of corresponding shape/configuration and having the general internal features of Figure 1 b.

For clarity, only the principal features of the internal mechanism features of the variations of Figures 3a and 3b are labelled. The cassette housing 300 houses medicament carrier blister strip 302 (see also Figure 2) coiled in a chamber 304 and fed about a guide wall 306 to the opening station 308 and beak 310. After the blister pack 302 is peeled into separate lid sheet 312 and base sheet 314, the lid sheet 312 is fed to a lid spool 316 in the lid spool chamber 316a, and the base sheet 314 to a base spool 318 in the base spool chamber 318a.

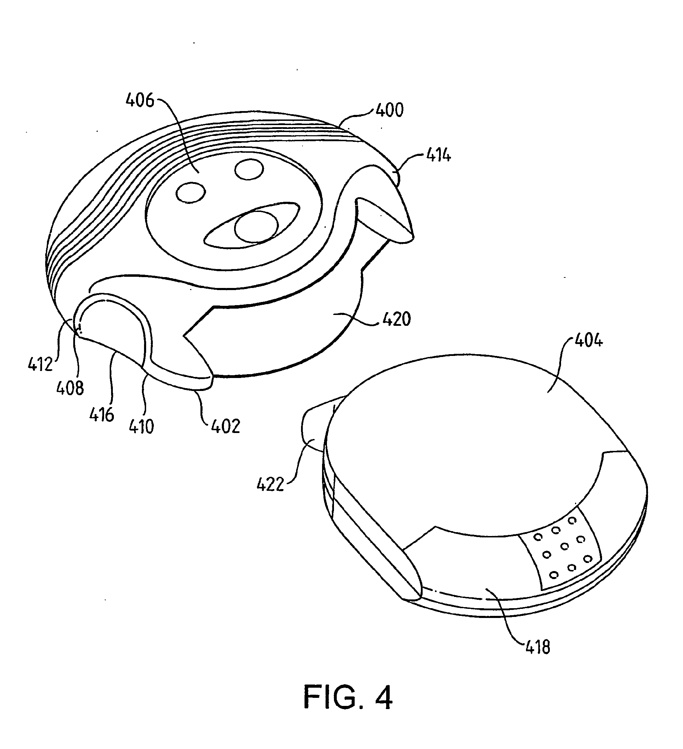

In the rest position, index ratchet 320 is in its 'locked position' in which it prevents movement of the blister strip 302. A nose 322 on the index ratchet 320 rests adjacent an already-opened medicament pocket 324 on the strip 302 hence halting further progression of the strip 302 through the mechanism. The index ratchet 320 is pivotally movable to a 'release position' in which it no longer contact the pocket 324 and thereby enables further travel of the blister strip 302 within the dispenser 300. Figure 4 shows a medicament dispenser in accord with the present invention, comprising a body 400, a holder 402, refill cassette 404 and electronic display 406. The holder 402 is shaped to fit snugly inside body 400 and is fixed to a point on the body (not shown) about which it rotates. Stops 408, 410 protrude from the holder 402 and prevent the holder 402 from rotating more than about 180° relative to the body 400. The stops 408, 410 also provide two defined positions of the holder 402 within the body 400. One position is defined by stop 408 meeting with body edge 412 and the other position defined by stop 410 meeting with body edge 414 when the holder has been rotated relative to the body. The area between stops 408 and 410 is shaped to form a thumb or finger grip 416 for the user of the device. The holder 402 forms a shell into which the refill cassette 404 snugly fits.

The refill cassette 404 comprises a shell containing the medicament carrier (not shown) and a mechanism for opening the carrier (not shown) for the medicament to be accessed. The refill cassette 404 has a raised portion 418 at one end on both sides along its width so that this part of the refill cassette 404 is at least the same depth as the part of the holder 420 which receives the refill cassette 404. This allows the position of the cassette 404 within the holder 402 to be fixed such that the ridge 418 preludes from the holder 402 but the rest of the cassette 404 is contained within the holder 402.

The refill cassette 404 also has a mouthpiece (not shown) and an indexing lever 422 for indexing the medicament carrier within the cassette 404.

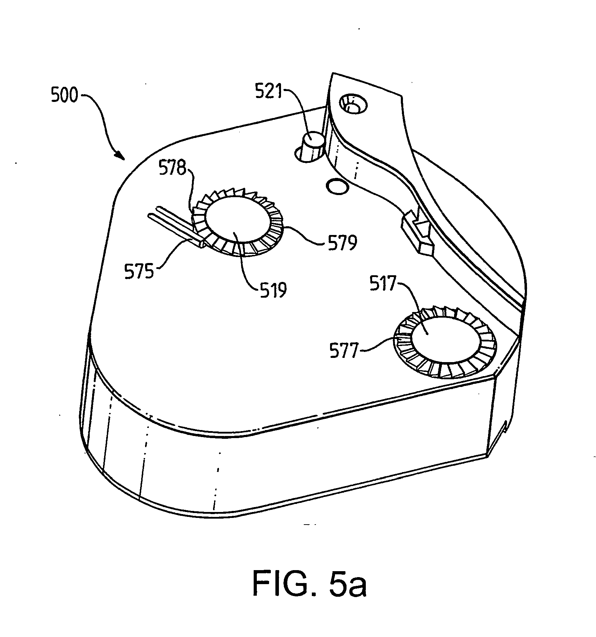

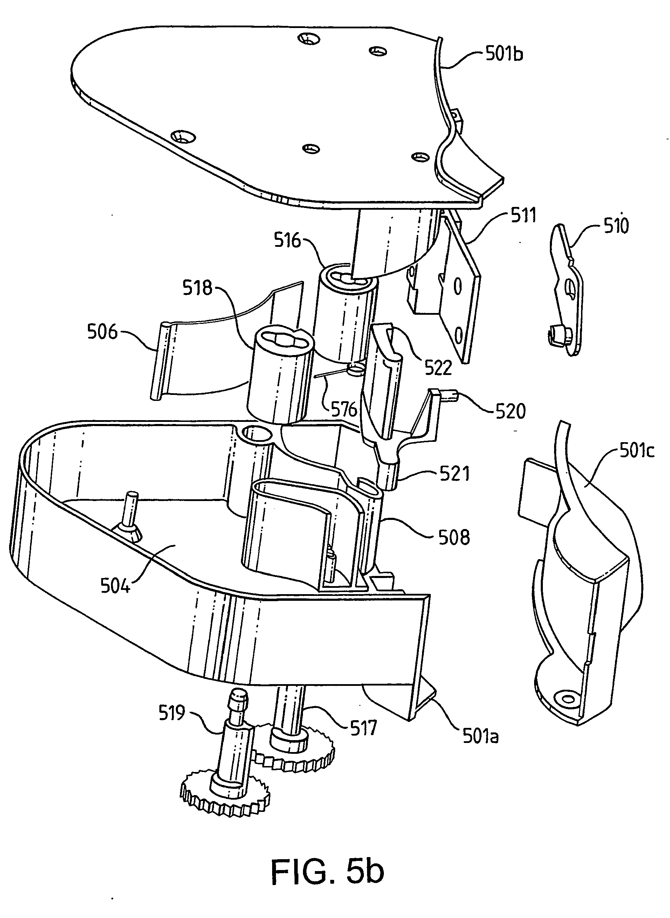

Figures. 5a and 5b illustrate a further refill cassette herein, in perspective and exploded views. The refill cassette housing of Figures 5a and 5b is shaped for ready receipt by a base unit (not shown) of suitable shape/configuration and having general mechanism features similar to those shown in Figure 1b. For clarity, only the principal features of the internal mechanism features of the variations of Figures 5a and 5b are labelled. The casing 500 of the cassette is in combination, formed of base 501 a, top 501 b and mouthpiece 501 c casing assembly elements. The casing 500 is generally shaped to receive a medicament carrier blister strip (not shown) that coils in chamber 504 and is fed about flexible guide membrane 506 to the opening station 508 for peeling open thereof. Adjacent to peeling station 508 there is also provided shutter 510 and outlet stack 511 components, which guide release of medicament to the mouthpiece 501 c. After the blister strip is peeled into its separate lid sheet and base sheet components (not shown), the lid sheet feeds onto lid spool 516 and the base sheet onto base spool 518.

There is further provided an index ratchet 520 that includes dogged end 522 for reversibly engaging blister strip and actuator lever 521, which protrudes from the cassette casing 500. Similarly to the earlier described embodiments of Figures 3a and 3b, the index ratchet 520 is movable from a 'locked position' in which it doggedly engages the blister strip to prevent its movement to a 'release position' in which it disengages the strip and thereby enables its travel within the dispenser 500 for peeling thereof.

The lid spool 516 and base spool 518 are provided with inner drive spindles 517, 519, each of which has a drive head with plural teeth 577, 579 for drivable engagement thereof by base unit drive elements (not shown). It will be appreciated that the respective drive heads have a generally flat profile, which in use (see Figure 5a) protrudes only slightly from the casing 500 thereby enabling ready receipt of the refill cassette by the base unit. The lid spool 516 is further provided with an index spring 576 and the base spool 518 further provided with a non-return ratchet 578 which co-operates with non-return leg 575 moulded into the casing body 501 a.

It may be appreciated that any of the parts of the dispenser or cassette which contact the medicament suspension may be coated with materials such as fluoropolymer materials (e.g. PTFE or FEP) which reduce the tendency of medicament to adhere thereto. Any movable parts may also have coatings applied thereto which enhance their desired movement characteristics. Frictional coatings may therefore be applied to enhance frictional contact and lubricants (e.g. silicone oil) used to reduce frictional contact as necessary.

The medicament dispenser of the invention is suitable for dispensing medicament, particularly for the treatment of respiratory disorders such as asthma and chronic obstructive pulmonary disease (COPD).

Appropriate medicaments may thus be selected from, for example, analgesics, e.g. codeine, dihydromorphine, ergotamine, fentanyl or morphine; anginal preparations, e.g., diltiazem; antiallergics, e.g., cromoglycate (eg as the sodium salt), ketotifen or nedocromil (eg as the sodium salt); antiinfectives e.g., cephalosporins, penicillins, streptomycin, sulphonamides, tetracyclines and pentamidine; antihistamines, e.g., methapyrilene; anti- inflammatories, e.g., beclomethasone (eg as the dipropionate ester), fluticasone (eg as the propionate ester), flunisolide, budesonide, rofleponide, mometasone (eg as the furoate ester), ciclesonide, triamcinolone (eg as the acetonide), 6α, 9α-difluoro-11β-hydroxy-16α-methyl-3-oxo-17α-propionyloxy-androsta-1,4-diene-17β-carbothioic acid S-(2-oxo-tetrahydro-furan-3-yl) ester or 6α, 9α-Difluoro-17α-[(2-furanylcarbonyl)oxy]-11β-hydroxy-16α-methyl-3-oxo-androsta-1,4-diene-17β-carbothtoic acid S-fluoromethyl ester, antitussives, e.g., noscapine; bronchodilators, e.g., albuterol (eg as free base or sulphate), salmeterol (eg as xinafoate), ephedrine, adrenaline, fenoterol (eg as hydrobromide), formoterol (eg as fumarate), isoprenaline, metaproterenol, phenylephrine, phenylpropanolamine, pirbuterol (eg as acetate), reproterol (eg as hydrochloride), rimiterol, terbutaline (eg as sulphate), isoetharine, tulobuterol or 4-hydroxy-7-[2-[[2-[[3-(2-phenylethoxy)propyl]sulfonyl]ethyl]amino]ethyl-2(3H)-benzothizaolone, PDE4 inhibitors eg cilomilast or roflumilast; leukotriene antagonists eg montelukast, pranlukast and zafirlukast; adenosine 2a agonists, e.g. 2R,3R,4S,5R)-2-[6-Amino-2-(1S-hydroxymethyl-2-phenyl-ethylamino)-purin-9-yl]-5-(2-ethyl-2H-tetrazol-5-yl)-tetrahydro-furan-3,4-diol (e.g. as maleate); α₄ integrin inhibitors e.g. (2S)-3-[4-({[4-(aminocarbonyl)-1-piperidinyl]carbonyl}oxy)phenyl]-2-[((2S)-4-methyl-2-[[2-(2-methylphenoxy) acetyl]amino}pentanoyl)amino] propanoic acid (e.g. as free acid or potassium salt), diuretics, e.g., amiloride; anticholinergics, e.g., ipratropium (eg as bromide), tiotropium, atropine or oxitropium; hormones, e.g., cortisone, hydrocortisone or prednisolone; xanthines, e.g., aminophylline, choline theophyllinate, lysine theophyllinate or theophylline; therapeutic proteins and peptides, e,g., insulin or glucagons. It will be clear to a person skilled in the art that, where appropriate, the medicaments may be used in the form of salts, (e.g., as alkali metal or amine salts or as acid addition salts) or as esters (e.g., lower alkyl esters) or as solvates (e.g., hydrates) to optimise the activity and/or stability of the medicament.

Preferred medicaments are selected from albuterol, salmeterol, fluticasone propionate and beclomethasone dipropionate and salts or solvates thereof, e.g., the sulphate of albuterol and the xinafoate of salmeterol.

Medicaments can also be delivered in combinations. Preferred formulations containing combinations of active ingredients contain salbutamol (e.g., as the free base or the sulphate salt) or salmeterol (e.g., as the xinafoate salt) or formoterol (eg as the fumarate salt) in combination with an anti-inflammatory steroid such as a beclomethasone ester (e.g., the dipropionate) or a fluticasone ester (e.g., the propionate) or budesonide. A particularly preferred combination is a combination of fluticasone propionate and salmeterol, or a salt thereof (particularly the xinafoate salt). A further Combination of particular interest is budesonide and formoterol (e.g. as the fumarate salt).

Generally, powdered medicament particles suitable for delivery to the bronchial or alveolar region of the lung have an aerodynamic diameter of less than 10 micrometers, preferably less than 6 micrometers. Other sized particles may be used if delivery to other portions of the respiratory tract is desired, such as the nasal cavity, mouth or throat. The medicament may be delivered as pure drug, but more appropriately, it is preferred that medicaments are delivered together with excipients (carriers) which are suitable for inhalation. Suitable excipients include organic excipients such as polysaccharides (i.e. starch, cellulose and the like), lactose, glucose, mannitol, amino acids, and maltodextrins, and inorganic excipients such as calcium carbonate or sodium chloride. Lactose is a preferred excipient.

Particles of the powdered medicament and/or excipient may be produced by conventional techniques, for example by micronisation, milling or sieving. Additionally, medicament and/or excipient powders may be engineered with particular densities, size ranges, or characteristics. Particles may comprise active agents, surfactants, wall forming materials, or other components considered desirable by those of ordinary skill.

The excipient may be included with the medicament via well known methods, such as by admixing, co-precipitating and the like. Blends of excipients and drugs are typically formulated to allow the precise metering and dispersion of the blend into doses. A standard blend, for example, contains 13000 micrograms lactose mixed with 50 micrograms drug, yielding an excipient to drug ratio of 260:1. Dosage blends with excipient to drug ratios of from 100:1 to 1:1 may be used. At very low ratios of excipient to drug, however, the drug dose reproducibility may become more variable.

It will be understood that the present disclosure is for the purpose of illustration only and the invention extends to modifications, variations and improvements thereto within the scope of the appended claims.

## Claims

1. A medicament dispenser for use with a medicament carrier (102; 302) having a plurality of pockets for containing medicament wherein said pockets are spaced along the length of and defined between two peelable sheets secured to each other, said dispenser having an internal mechanism for accessing said medicament contained within said medicament carrier, said mechanism comprising,
a) an opening station (108; 308; 508) for receiving a pocket of said medicament carrier;
b) peeling means (116, 118; 316, 318; 516, 518) positioned to engage a base sheet (114; 314) and a lid sheet (112; 312) of a pocket which has been received in said opening station for peeling apart such a base, sheet (134, 138, 140) and lid sheet, to open such a pocket, said peeling means including lid driving means (134, 138, 140) for pulling apart a lid sheet and a base sheet of a pocket that has been received at said opening station;
c) an outlet, positioned to be in communication with an opened pocket through which a user can remove medicament from such an opened pocket; and
d) indexing means for indexing in communication with said outlet, pockets of a medicament carrier in use with said medicament dispenser,
**characterised in that** said indexing means comprises an index ratchet (120; 320; 520) which is movable between a locked position in which said ratchet engages said medicament carrier to prevent movement thereof and a release position in which the ratchet disengages from the medicament carrier to allow movement thereof for peeling open of a blister pocket.

2. A medicament dispenser according to claim 1, wherein actuation of said medicament dispenser actuates said lid driving means and disengages said index ratchet from the medicament carrier.

3. A medicament dispenser according to either of claims 1 or 2, wherein the index ratchet comprises a ratchet arm.

4. A medicament dispenser according to claim 3, wherein said ratchet arm is pivotally mounted on the dispenser.

5. A medicament dispenser according to any of claims 1 to 4, wherein in the locked position the index ratchet engages a blister pocket of said medicament carrier and in the release position said pocket is disengaged.

6. A medicament dispenser according to claim 5, wherein the index ratchet is positioned to cam in and out of engagement with said blister pocket (124; 324) of the medicament carrier.

7. A medicament dispenser according to any of claims 1 to 6, further comprising an indexing lever (132; 422; 521) for actuating said dispenser.

8. A medicament dispenser according to claim 7, wherein said indexing lever comprises cam means (150) for moving said index ratchet between locked and release positions, such that actuation of said lever from a stop position releases said medicament carrier for peeling thereof.

9. A medicament dispenser according to either of claims 7 or 8, wherein said indexing lever comprises a lever ratchet (136) for engaging said lid driving means.

10. A medicament dispenser according to any one of the preceding claims, wherein said lid driving means comprises an index gear (138) and a drive gear (140) which are interconnected so that the rotation of one correlates with the rotation of the other.

11. A medicament dispenser according to any one of the preceding claims, wherein said lid driving means comprises a wheel (116; 316; 516) on which the lid sheet is wound up.

12. A medicament dispenser according to claim 11, wherein said lid sheet wheel has an effective winding surface the diameter of which increases after every use of the dispenser

13. A medicament dispenser according to any of claims 7 to 12 further comprising a lever stop means to limit the extent of movement of said index lever and thereby said lid driving means, in order to control the length of medicament carrier peeled by said peeling means.

14. A medicament dispenser according to claim 13 further comprising compensating means positioned between said opening station and said lid sheet wheel for reducing the length of lid sheet therebetween to compensate for any increase in the diameter of said effective winding surface of the lid sheet wheel during use of the dispenser.

15. A medicament dispenser according to any one of the preceding claims, wherein the internal mechanism for accessing the said medicament contained within said medicament carrier is housed within a cassette (100; 300; 404; 500).

16. A medicament dispenser according to claim 15 comprising,
a body (400).
a holder (402), shaped to fit within said body and movable relative to said body; and receivable by said holder, said cassette containing said medicament carrier.

17. A medicament dispenser according to claim 16, wherein movement of said holder relative to said body results in movement of said cassette between a first position and a second position such that the cassette is reversibly removable from the holder when the cassette is in the second position.

18. A medicament dispenser according to claim 17, wherein the first position comprises a dispensing position.

19. A medicament dispenser according to claim 18, wherein the second position comprises a non-dispensing position.

20. A medicament dispenser according to any of claims 16 to 19, wherein the holder and body include attaching means to attach the holder to the body.

21. A medicament dispenser according to any of claims 15 to 20 when appended to claim 7, wherein the cassette additionally comprises the indexing lever.

22. A medicament dispenser according to any of claims 15 to 21, wherein the cassette additionally comprises a mouthpiece.

23. A medicament dispenser according to any one of the preceding claims additionally comprising a medicament carrier comprising medicament in powdered or solid (e.g. tablet) form.

## Patentansprüche

1. Medikamentenspender zur Verwendung mit einem Medikamententräger (102; 302) mit einer Vielzahl von Taschen zur Aufnahme eines Medikaments, wobei die Taschen entlang der Länge von zwei ablösbaren Bögen, die aneinander befestigt sind, beabstandet sind, und sie zwischen den Bögen definiert werden, wobei der Spender einen inneren Mechanismus zum Zugriff auf das innerhalb des Medikamententrägers enthaltene Medikament aufweist, wobei der Mechanismus umfasst:
a) eine Öffnungsstelle (108; 308; 508) zum Empfangen einer Tasche des Medikamententrägers;
b) eine Ablöse-Einrichtung (116, 118; 316, 318; 516, 518), die positioniert ist, um einen Grundbogen (114; 314) und einen Deckbogen (112; 312) von einer Tasche in Eingriff zu nehmen, die in der Öffnungsstelle zum Lösen eines derartigen Grundbogens und Deckbogens voneinander empfangen wurde, um eine derartige Tasche zu öffnen, wobei die Ablöse-Einrichtung eine Deckelantriebseinrichtung (134, 138, 140) umfasst, zum Auseinanderziehen eines Deckbogens und eines Grundbogens einer Tasche, die an der Öffnungsstelle empfangen wurde;
c) einen Auslass, der positioniert ist, um sich mit einer geöffneten Tasche in Verbindung zu befinden, durch den ein Nutzer ein Medikament aus einer derartigen geöffneten Tasche entnehmen kann; und
d) eine Indizierungseinrichtung zum Indizieren, in Verbindung mit dem Auslass, von Taschen eines mit dem Medikamentenspender verwendeten Medikamententrägers;
**dadurch gekennzeichnet, dass** die Indizierungseinrichtung eine Index-Ratsche (120; 320; 520) umfasst, die zwischen einer verriegelten Position, in der die Ratsche den Medikamententräger in Eingriff nimmt, um eine Bewegung desselben zu verhindern, und einer Freigabeposition bewegbar ist, in der die Ratsche von dem Medikamententräger außer Eingriff gelangt, um eine Bewegung desselben zum Aufziehen einer Blistertasche zuzulassen.

2. Medikamentenspender nach Anspruch 1, bei dem eine Betätigung des Medikamentenspenders die Deckelantriebseinrichtung betätigt und die Index-Ratsche von dem Medikamententräger außer Eingriff bringt.

3. Medikamentenspender nach Anspruch 1 oder 2, bei dem die Index-Ratsche einen Ratschen-Arm umfasst.

4. Medikamentenspender nach Anspruch 3, bei dem der Ratschen-Arm an dem Spender schwenkbar angebracht ist.

5. Medikamentenspender nach einem der Ansprüche 1 bis 4, bei dem die Index-Ratsche in der verriegelten Position eine Blister-Tasche des Medikamententrägers in Eingriff nimmt, und in der Freigabeposition die Tasche außer Eingriff ist.

6. Medikamentenspender nach Anspruch 5, bei dem die Index-Ratsche so positioniert ist, dass sie mit der Blister-Tasche (124; 324) des Medikamententrägers in und außer Eingriff gelangt.

7. Medikamentenspender nach einem der Ansprüche 1 bis 6, ferner mit einem Indizierungshebel (132; 422; 521) zum Betätigen des Spenders.

8. Medikamentenspender nach Anspruch 7, bei dem der Indizierungshebel eine Eingriffseinrichtung (150) umfasst, zum Bewegen der Index-Ratsche zwischen der verriegelten Position und der Freigabeposition, derart, dass eine Betätigung des Hebels aus einer Halteposition den Medikamententräger zum Abziehen desselben freigibt.

9. Medikamentenspender nach Anspruch 7 oder 8, bei dem der Indizierungshebel eine Hebel-Ratsche (136) umfasst, zum Eingriff mit der Deckelantriebseinrichtung.

10. Medikamentenspender nach einem der vorhergehenden Ansprüche, bei dem die Deckelantriebseinrichtung ein Index-Zahnrad (138) und ein Antriebszahnrad (140) umfasst, die miteinander verbunden sind, so dass die Drehung des einen mit der Drehung des anderen korreliert.

11. Medikamentenspender nach einem der vorhergehenden Ansprüche, bei dem die Deckelantriebseinrichtung ein Rad (116; 316; 516) umfasst, auf dem der Deckbogen aufgewickelt ist.

12. Medikamentenspender nach Anspruch 11, bei dem das Deckbogen-Rad eine effektive Wicklungsoberfläche aufweist, deren Durchmesser nach jeder Nutzung des Spenders zunimmt.

13. Medikamentenspender nach einem der Ansprüche 7 bis 12, ferner mit einer Hebel-Halteeinrichtung, um das Ausmaß der Bewegung des Index-Hebels, und **dadurch** der Deckelantriebseinrichtung, zu beschränken, um die Länge des durch die Ablöse-Einrichtung abgezogenen Medikamententrägers zu steuern.

14. Medikamentenspender nach Anspruch 13, ferner mit einer Kompensationseinrichtung, die zwischen der Öffnungsstelle und dem Deckbogen-Rad positioniert ist, zur Verringerung der Länge von Deckbogen dazwischen, um jegliche Zunahme des Durchmessers der effektiven Wicklungsoberfläche des Deckbogen-Rads während der Nutzung des Spenders zu kompensieren.

15. Medikamentenspender nach einem der vorhergehenden Ansprüche, bei dem der innere Mechanismus zum Zugriff auf das innerhalb des Medikamententrägers enthaltene Medikament innerhalb einer Kassette (100; 300; 404; 500) aufgenommen ist.

16. Medikamentenspender nach Anspruch 15, mit:
einem Körper (400);
einem Halter (402), der so geformt ist, dass er in den Körper passt und relativ zu dem Körper bewegbar ist; und
durch den Halter empfangbar ist, wobei die Kassette den Medikamententräger enthält.

17. Medikamentenspender nach Anspruch 16, bei dem eine Bewegung des Halters relativ zu dem Körper in einer Bewegung der Kassette zwischen einer ersten Position und einer zweiten Position resultiert, derart, dass die Kassette von dem Halter reversibel entfernbar ist, wenn die Kassette in der zweiten Position ist.

18. Medikamentenspender nach Anspruch 17, bei dem die erste Position eine Abgabeposition umfasst.

19. Medikamentenspender nach Anspruch 18, bei dem die zweite Position eine Nicht-Abgabeposition umfasst.

20. Medikamentenspender nach einem der Ansprüche 16 bis 19, bei dem der Halter und Körper Befestigungsmittel umfassen, um den Halter an dem Körper anzubringen.

21. Medikamentenspender nach einem der Ansprüche 15 bis 20, wenn abhängig von Anspruch 7, bei dem die Kassette zusätzlich den Index-Hebel umfasst.

22. Medikamentenspender nach einem der Ansprüche 15 bis 21, bei dem die Kassette zusätzlich ein Mundstück aufweist.

23. Medikamentenspender nach einem der vorhergehenden Ansprüche, zusätzlich mit einem Medikamententräger, der ein Medikament in Pulverform oder fester (z.B. Tabletten) Form umfasst.

## Revendications

1. Distributeur de médicaments destiné à être utilisé avec un porte-médicament (102 ; 302) possédant une pluralité de pochettes destinées à contenir un médicament dans lequel lesdites pochettes sont espacées sur la longueur de et définies entre deux feuilles pouvant être décollées fixées l'une à l'autre, ledit distributeur possédant un mécanisme interne destiné à accéder audit médicament contenu à l'intérieur dudit porte-médicament, ledit mécanisme comprenant,
a) une station d'ouverture (108 ; 308 ; 508) destinée à recevoir une pochette dudit porte-médicament ;
b) des moyens de décollage (116, 118 ; 316, 318 ; 516, 518) positionnés pour entrer en prise avec une feuille de base (114 ; 314) et une feuille de couvercle (112 ; 312) d'une pochette qui a été reçue dans ladite station d'ouverture pour décoller l'une de l'autre de telles feuille de base et feuille de couvercle, pour ouvrir une telle pochette, lesdits moyens de décollage comprenant des moyens d'entraînement de couvercle (134, 138, 140) destinés à séparer l'une de l'autre en tirant une feuille de couvercle et une feuille de base d'une pochette qui a été reçue au niveau de ladite station d'ouverture ;
c) une sortie, positionnée pour être en communication avec une pochette ouverte à travers laquelle un utilisateur peut retirer un médicament d'une telle pochette ouverte ; et
d) des moyens d'indexage destinés à indexer en communication avec ladite sortie, des pochettes d'un porte-médicament en fonctionnement avec ledit distributeur de médicaments,
**caractérisé en ce que** lesdits moyens d'indexage comprennent un cliquet d'index (120 ; 320 ; 520) qui est mobile entre une position verrouillée dans laquelle ledit cliquet entre en prise avec ledit porte-médicament pour empêcher le mouvement de celui-ci et une position de libération dans laquelle le cliquet se disjoint du porte-médicament pour permettre le mouvement de celui-ci pour l'ouverture par décollage d'une pochette-coque.

2. Distributeur de médicaments selon la revendication 1, dans lequel l'actionnement dudit distributeur de médicaments actionne lesdits moyens d'entraînement de couvercle et disjoint ledit cliquet d'index du porte-médicament.

3. Distributeur de médicaments selon la revendication 1 ou 2, dans lequel le cliquet d'index comprend un bras de cliquet.

4. Distributeur de médicaments selon la revendication 3, dans lequel ledit bras de cliquet est monté de façon pivotante sur le distributeur.

5. Distributeur de médicaments selon l'une quelconque des revendications 1 à 4, dans lequel, dans la position verrouillée, le cliquet d'index entre en prise avec une pochette-coque dudit porte-médicament et, dans la position de libération, ladite pochette est disjointe.

6. Distributeur de médicaments selon la revendication 5, dans lequel le cliquet d'index est positionné pour entrer, par came, en prise avec, et hors de prise de, ladite pochette-coque (124 ; 324) du porte-médicament.

7. Distributeur de médicaments selon l'une quelconque des revendications 1 à 6, comprenant en outre un levier d'indexage (132 ; 422 ; 521) destiné à actionner ledit distributeur.

8. Distributeur de médicaments selon la revendication 7, dans lequel ledit levier d'indexage comprend des moyens de came (150) destinés à déplacer ledit cliquet d'index entre des positions verrouillée et de libération, de sorte que l'actionnement dudit levier à partir d'une position d'arrêt libère ledit porte-médicament pour le décollage de celui-ci.

9. Distributeur de médicaments selon la revendication 7 ou 8, dans lequel ledit levier d'indexage comprend un cliquet de levier (136) destiné à entrer en prise avec lesdits moyens d'entraînement de couvercle.

10. Distributeur de médicaments selon l'une quelconque des revendications précédentes, dans lequel lesdits moyens d'entraînement de couvercle comprennent un engrenage d'index (138) et un engrenage d'entraînement (140) qui sont reliés mutuellement de sorte que la rotation de l'un se fasse en corrélation avec la rotation de l'autre.

11. Distributeur de médicaments selon l'une quelconque des revendications précédentes, dans lequel lesdits moyens d'entraînement de couvercle comprennent une roue (116 ; 316 ; 516) sur laquelle est enroulée la feuille de couvercle.

12. Distributeur de médicaments selon la revendication 11, dans lequel ladite roue de feuille de couvercle possède une surface d'enroulement efficace dont le diamètre augmente après chaque utilisation du distributeur.

13. Distributeur de médicaments selon l'une quelconque des revendications 7 à 12, comprenant en outre un moyen de butée de levier pour limiter l'étendue de mouvement dudit levier d'index et ainsi desdits moyens d'entraînement de couvercle, afin de contrôler la longueur de porte-médicament décollée par lesdits moyens de décollage.

14. Distributeur de médicaments selon la revendication 13, comprenant en outre des moyens de compensation positionnés entre ladite station d'ouverture et ladite roue de feuille de couvercle pour réduire la longueur de feuille de couvercle entre celles-ci pour compenser toute augmentation du diamètre de ladite surface d'enroulement efficace de la roue de feuille de couvercle au cours de l'utilisation du distributeur.

15. Distributeur de médicaments selon l'une quelconque des revendications précédentes, dans lequel le mécanisme interne destiné à accéder audit médicament contenu à l'intérieur dudit porte-médicament est logé à l'intérieur d'une cassette (100 ; 300 ; 404 ; 500).

16. Distributeur de médicaments selon la revendication 15 comprenant,
un corps (400) ;
un élément de support (402), formé pour aller à l'intérieur dudit corps et mobile par rapport audit corps ; et
recevable par ledit élément de support, ladite cassette contenant ledit porte-médicament.

17. Distributeur de médicaments selon la revendication 16, dans lequel le mouvement dudit élément de support par rapport audit corps entraîne le mouvement de ladite cassette entre une première position et une seconde position de sorte que la cassette soit amovible de façon réversible à partir de l'élément de support lorsque la cassette est dans la seconde position.

18. Distributeur de médicaments selon la revendication 17, dans lequel la première position comprend une position de distribution.

19. Distributeur de médicaments selon la revendication 18, dans lequel la seconde position comprend une position de non-distribution.

20. Distributeur de médicaments selon l'une quelconque des revendications 16 à 19, dans lequel l'élément de support et le corps comprennent des moyens de fixation pour fixer l'élément de support au corps.

21. Distributeur de médicaments selon l'une quelconque des revendications 15 à 20 lorsqu'elles dépendent de la revendication 7, dans lequel la cassette comprend de plus le levier d'indexage.

22. Distributeur de médicaments selon l'une quelconque des revendications 15 à 21, dans lequel la cassette comprend de plus un embout buccal.

23. Distributeur de médicaments selon l'une quelconque des revendications précédentes, comprenant de plus un porte-médicament comprenant un médicament en poudre ou solide (par exemple un comprimé).
